# EUROPÄISCHE PATENTSCHRIFT

(11) **EP 3 286 294 B1**
(45) Veröffentlichungstag und Bekanntmachung des Hinweises auf die Patenterteilung: **05.10.2022**
(21) Anmeldenummer: 16721095.4
(22) Anmeldetag: 19.04.2016
(51) Int. Cl.: A23L 2/52, A23L 2/72, A23L 2/80, C12H 1/07, C12H 1/16, C12H 1/056

(54) **VERFAHREN UND VORRICHTUNG ZUR STABILISIERUNG POLYPHENOLHALTIGER FLÜSSIGKEITEN**
METHOD AND DEVICE FOR STABILIZING POLYPHENOL-CONTAINING LIQUIDS
PROCÉDÉ ET DISPOSITIF POUR STABILISER DES LIQUIDES CONTENANT DU POLYPHÉNOL

(30) Priorität: 23.04.2015 DE 102015106258
(43) Veröffentlichungstag der Anmeldung: 28.02.2018
(73) Patentinhaber: KHS GmbH, 44143 Dortmund (DE)
(72) Erfinder: MONZEL, Alois, 67591 Mörstadt (DE)
(86) Internationale Anmeldenummer: PCT/EP2016/058635
(87) Internationale Veröffentlichungsnummer: WO 2016/169924

(56) Entgegenhaltungen:
- WO-A1-2010/142364
- WO-A2-2009/124649
- CH-A- 503 319
- DE-A1- 10 158 448
- DE-A1- 19 642 229
- DE-T2- 69 512 565

## Beschreibung

### Technisches Gebiet

Die Erfindung bezieht sich auf ein Verfahren zur Stabilisierung von zumindest eine Art von Polyphenol enthaltenden Flüssigkeiten sowie auf eine Vorrichtung zur Durchführung des Verfahrens.

### Stand der Technik

Flüssigkeiten, insbesondere Getränke wie zum Beispiel Wein oder Bier unterliegen häufig einem zeitlich abhängigen Trübungsprozess, wobei die Trübungseigenschaften oder das Trübungspotential durch das Vorhandensein bestimmter Stoffe, insbesondere durch in der Flüssigkeit enthaltene Polyphenole beeinflusst werden. Polyphenole sind aromatische Verbindungen, die zu den sekundären Pflanzenstoffen gezählt werden und als bioaktive Substanzen in unterschiedlichsten Formen natürlich vorkommen. Die Trübung polyphenolhaltiger Flüssigkeiten wird beispielsweise durch chemische und/oder physikalische Reaktion der polyphenolischen Verbindungen mit im Getränk enthaltenen Proteinen ausgelöst. Obwohl in den meisten Fällen die Haltbarkeit von Flüssigkeiten oder Getränken durch eine auftretende kolloidale Trübung nicht beeinträchtigt wird, ist jedoch für den Verbraucher die Trübung von Getränken inakzeptabel. Es ist daher vor allem für die Getränkeindustrie bzw. für Brauereien ein besonderes Anliegen, die kolloidale Stabilität, also die Trübungsfreiheit von Getränken zu verbessern.

Um der durch Polyphenole hervorgerufenen Trübung in Flüssigkeiten, insbesondere in Getränken entgegenzuwirken, werden Polyphenole bzw. polyphenolische Verbindungen aus den Getränken abgereichert oder entfernt. Dieser Prozess der Entfernung oder der Reduzierung der polyphenolischen Verbindungen und/oder bestimmten Proteinfraktionen aus Getränken wird auch als Stabilisierung der Getränke bezeichnet.

Zum Beispiel sind zur Entfernung von Polyphenolen aus Bier aus dem Stand der Technik diverse mehrstufige Verfahren bekannt. Hierbei werden dem Bier vor der eigentlichen Entfernung der polyphenolischen Verbindungen zunächst Hefen und Trübstoffe mittels Filtration entzogen. Im Rahmen des eigentlichen Stabilisierungsprozesses wird das Bier dann zunächst mit einem Stabilisierungsmittel in Kontakt gebracht, welches einen bevorzugten Reaktionspartner für die polyphenolischen Verbindungen und/oder bestimmten Proteinfraktionen darstellt. Hierbei werden Verfahren mit verlorenen Stabilisierungsmitteln von Verfahren mit der Möglichkeit der Regeneration des Stabilisierungsmittels unterschieden. Als Stabilisierungsmittel findet in der Praxis häufig Polyvinylpolypyrrolidon Verwendung, wobei die Polyphenole an das Polyvinylpolypyrrolidon gebunden werden. Die an Polyvinylpolypyrrolidon gebundenen Polyphenole können anschließend beispielsweise mittels Filtration aus dem Bier entfernt werden, wodurch die Bildung von Trübungen auch bei längerer Lagerdauer des Bieres nahezu vollständig verhindert, zumindest aber stark reduziert werden kann.

Ebenfalls ist es bekannt, Polyphenole durch Enzyme aus dem Bier zu entfernen, zumindest aber deren Anteil im Bier bzw. im Getränk deutlich zu reduzieren.

Dafür geeignete Enzyme sind beispielsweise Pepsin, Papsin, Brewers Clarex. Der Grad der Entfernung der Polyphenole aus Flüssigkeiten wie Bier ist von einer Vielzahl von Faktoren abhängig. Von besonderer Bedeutung hierbei sind die Menge des zugesetzten Stabilisierungsmittels, die Anzahl der freien Adsorptionsstellen des Stabilisierungsmittels und die Länge der Kontaktzeit der Flüssigkeit mit dem Stabilisierungsmittel. Dabei ist es bekannt, durch geeignete Wahl und Auslegung der Komponenten einer Stabilisierungsvorrichtung beispielsweise hinsichtlich Tankvolumen, Strömungsgeschwindigkeiten usw., die gesamte Stabilisierungsvorrichtung derart auszulegen, dass hinreichende Kontaktzeiten zwischen dem Stabilisierungsmittel und den zu entfernenden Bestandteilen erzielt werden können und somit auch hinreichende Stabilisierungsgrade erreicht werden.

Ein derartiges Verfahren ist aus DE 196 42 229 A1 bekannt. Dabei ist im Detail vorgesehen, zur Bestimmung der phenolartigen Verbindungen in Flüssigkeiten ein flüssiges Reagenz aus Polyphenylpyrrolidon oder deren Copolymeren einzusetzen.

Auch ist zu berücksichtigen, dass die stabilisierende Wirkung des Stabilisierungsmittels Polyvinylpolypyrrolidon oder anderer geeigneter Stabilisierungsmittel mit zunehmender Kontakt- oder Einwirkzeit abnimmt. Dies ist darauf zurückzuführen, dass die vorhandenen Adsorptionsstellen des Stabilisierungsmittels immer stärker gesättigt werden. In diesem Zusammenhang ist es bekannt, während der Zufuhr von zu stabilisierendem Bier diesem näherungsweise zeitgleich frisches Stabilisierungsmittel zuzusetzen, so dass stets eine ausreichende Anzahl von unbesetzten Adsorptionsstellen vorhanden ist. Das Stabilisierungsmittel Polyvinylpolypyrrolidon wird vorzugsweise regeneriert, und zwar beispielsweise mittels Natronlauge.

Hat das Bier nun den gewünschten Stabilisierungsgrad erreicht, so werden das Stabilisierungsmittel und das Bier wieder voneinander getrennt, um eine Überstabilisierung bzw. die mit einer solchen Überstabilisierung verbundenen erhöhten Kosten zu vermeiden. Zur Trennung des Stabilisierungsmittels vom Bier werden in der Praxis Filtereinheiten, beispielsweise sogenannte Anschwemmfilter oder andere geeignete Verfahren verwendet, wie sie dem Fachmann allgemein bekannt sind. Bei der Anschwemmfiltration wird das der zu stabilisierenden Flüssigkeit zugesetzte Stabilisierungsmittel dadurch entzogen, dass dieses gegen die Filterfläche angeschwemmt und von der Filterfläche zurückgehalten wird, wohingegen die Flüssigkeit die Filterfläche passieren kann. Zur Bestimmung des jeweils erreichten Ist-Stabilisierungsgrades wurden eine Vorrichtung und ein Verfahren nach der DE101 58 448 A1 bekannt. Mehr im Detail wird dabei der Ist-Stabilisierungsgrad durch die Verwendung einer Fotometereinheit bestimmt, welche die Veränderungen eines, durch die zu stabilisierende Flüssigkeit dringenden elektromagnetischen Welle erfasst und auswertet.

Zur Anschwemmfiltration werden Horizontalfilter und sogenannte Kerzenfilter eingesetzt, wie sie dem Fachmann allgemein bekannt sind.

Die DE 10 2011 014 184 A1 offenbart beispielsweise eine Vorrichtung und ein Verfahren zur Stabilisierung einer Flüssigkeit, insbesondere Bier, wobei das Gemisch aus Flüssigkeit und Stabilisierungsmittel zum Entfernen des Stabilisierungsmittels aus der stabilisierten Flüssigkeit einer ersten und einer zweiten Filtereinheit zugeführt wird. Die erste und zweite Filtereinheit werden dabei in zeitlich zueinander versetzten Betriebsphasen betrieben, wobei jeder Filtereinheit nach einer Regenerations- und Reinigungsphase erneut unstabilisierte Flüssigkeit mit zugesetztem Stabilisierungsmittel zugeführt wird.

Ein grundsätzliches Problem bei den bekannten Lösungen zur Stabilisierung polyphenolhaltiger Flüssigkeiten ist, dass der Gehalt bzw. die enthaltene Gesamtmenge an Polyphenolen in der Flüssigkeit, insbesondere in Getränken, während oder bei der Stabilisierung nicht ermittelt werden kann. Das Stabilisierungsmittel kann daher nicht mengenproportional zu der zu stabilisierenden Flüssigkeit zugegeben werden. Da in der Praxis eine Unterstabilisierung der Getränke auf jeden Fall vermieden werden soll, ist es gängige Praxis, dass das Stabilisierungsmittel in Mengen zugesetzt wird, die in jedem Fall für einen gewünschten Grad an Stabilisierung ausreichen. Diese hohe Dosierung oder Überdosierung des Stabilisierungsmittels führt zu unerwünschten und erhöhten Kosten bei der Getränkestabilisierung, so dass es trotz der aus dem Stand der Technik bekannten Lösungen einen Bedarf gibt, die Verfahren und Vorrichtungen zur Stabilisierung von Flüssigkeiten im Hinblick auf eine wirtschaftliche Anwendung weiter zu entwickeln.

### Darstellung der Erfindung

Aufgabe der vorliegenden Erfindung ist es daher ein Verfahren sowie eine Vorrichtung zur Verfügung zu stellen, die eine möglichst kostengünstige und materialsparende Stabilisierung polyphenolhaltiger Flüssigkeiten erlaubt. Diese Aufgabe wird erfindungsgemäß durch das Verfahren gemäß unabhängigem Anspruch 1 sowie durch die Vorrichtung gemäß unabhängigem Anspruch 15 gelöst. Weitere vorteilhafte Aspekte, Details und Ausgestaltungen der Erfindung ergeben sich aus den abhängigen Ansprüchen, der Beschreibung sowie den Zeichnungen.

Die vorliegende Erfindung macht sich die überraschende Erkenntnis zunutze, dass es eine spektroskopische Untersuchung von polyphenolhaltigen Flüssigkeiten in Verbindung mit einer Analyse der dabei erfassten elektromagnetischen Wellen, insbesondere der dabei auftretenden Emissionslinien oder Absorptionslinien erlaubt, auf den Polyphenolgehalt der untersuchten Flüssigkeit zu schließen.

Dieses gelingt insbesondere dann, wenn die erfassten elektromagnetischen Wellen mit Referenz- oder Vergleichsspektren verglichen werden.

Nachfolgend soll unter dem Begriff "Spektrum" das Ergebnis einer spektroskopischen Untersuchung, mehr im Detail also die dabei erfassten elektromagnetischen Wellen verstanden werden.

Die vorliegende Erfindung stellt ein Verfahren zur Stabilisierung von Flüssigkeiten zur Verfügung, die zumindest eine Art von Polyphenol enthaltenden.

Dabei umfasst das das Verfahren wenigstens die im Folgenden beschriebenen aufeinanderfolgenden Schritte a) bis g).

In Schritt a) des Verfahrens wird zunächst mittels einer Bereitstellungs- und Zuführeinheit eine zumindest eine Art von Polyphenol enthaltende Flüssigkeit bereitgestellt und in einem darauffolgenden Schritt b) wird wenigstens ein Spektrum der Flüssigkeit mittels zumindest einer vorgesehenen und dafür eingerichteten Messvorrichtung aufgenommen. In einem weiteren Schritt c) des Verfahrens wird mittels einer vorgesehenen und dafür eingerichteten Vergleichs- und Auswerteeinheit eine quantitative Menge der in der Flüssigkeit enthaltenen zumindest einen Art von Polyphenol ermittelt, wobei die Vergleichs- und Auswerteeinheit das in Schritt b) aufgenommene wenigstens eine Spektrum der Flüssigkeit mit wenigstens einem Referenzspektrum vergleicht und aus dem Vergleich durch geeignete Vergleichsoperationen einen Wert für die quantitative Menge der in der Flüssigkeit enthaltenen zumindest einen Art von Polyphenol errechnet.

In Folge des Schrittes c) wird sodann in einem Schritt d) des Verfahrens mittels einer Dosiereinheit eine bestimmte Menge eines in einer Flüssigkeit unlöslichen Stabilisierungsmittels zu der Flüssigkeit zugesetzt, wobei die Menge an Stabilisierungsmittel in Abhängigkeit des in Schritt c) errechneten Wertes für die quantitative Menge an Polyphenol in der Flüssigkeit bestimmt wird und wobei das Stabilisierungsmittel zum Binden von wenigstens einer Art von Polyphenol ausgebildet ist.

In einem weiteren Schritt e) des Verfahrens wird das Gemisch aus Flüssigkeit und Stabilisierungsmittel zumindest einer vorgesehenen Filtereinheit zugeführt.

In Schritt f) des Verfahrens wird das wenigstens teilweise mit Polyphenol beladene Stabilisierungsmittel mittels der Filtereinheit abgetrennt und schließlich wird in Schritt g) die von der wenigstens einen Art von Polyphenol abgereicherte, stabilisierte Flüssigkeit mittels einer Abführleitung abgeführt.

Besonders vorteilhaft kann über die Messung bzw. Aufnahme mindestens eines Spektrums der zu stabilisierenden Flüssigkeit mittels des erfindungsgemäßen Verfahrens ein Wert für die quantitative Menge der in der Flüssigkeit enthaltenen zumindest einen Art von Polyphenol errechnet werden. Dies ist gleichbedeutend damit, dass die in der Flüssigkeit enthaltene quantitative Menge an Polyphenol bzw. der in der Flüssigkeit enthaltene Anteil an Polyphenol bestimmt werden kann, wobei unter der in der Flüssigkeit enthaltenen quantitativen Menge an Polyphenol vorliegend auch der Gehalt bzw. Gesamtgehalt an Polyphenol in der Flüssigkeit oder die enthaltene Polyphenol-Stoffmenge oder die Konzentration an Polyphenol verstanden wird. Aufgrund dieser mit dem erfindungsgemäßen Verfahren gegebenen Möglichkeit, die Gesamtmenge an Polyphenol in der Flüssigkeit zu bestimmen, kann das Stabilisierungsmittel insbesondere vorteilhaft in Abhängigkeit der ermittelten Polyphenolmenge bzw. des ermittelten Polyphenolgehalts zur Flüssigkeit zugegeben werden, d. h. das Stabilisierungsmittel kann mengenproportional zugesetzt werden, wodurch eine Überdosierung vermieden und dadurch eine merkliche Kosteneinsparung erreicht werden kann.

Die Ermittlung der quantitativen Menge an Polyphenol in der Flüssigkeit umfasst erfindungsgemäß einen Vergleich des aufgenommenen zumindest einen Spektrums der Flüssigkeit mit einem Referenzspektrum und eine anschließende geeignete Auswertung der Vergleichsdaten.

**Als Referenzspektrum wird vorliegend bevorzugt zumindest ein Spektrum der entsprechend zu stabilisierenden bzw. zu untersuchenden, jedoch polyphenolfreien Flüssigkeit verwendet.**

**Alternativ oder aber auch ergänzend kann als Referenzspektrum auch ein Spektrum einer Flüssigkeit verwendet werden, die einen von Null unterschiedlichen aber bekannten Polyphenolgehalt aufweist.**

Besonders vorteilhaft ist es, wenn mehrere Spektren verwendet werden, die jeweils für Flüssigkeiten mit unterschiedlichen Polyphenolgehalten gelten, bzw. erstellt wurden.

**Das mindestens eine Referenzspektrum ist daher besonders gut Vergleichsanalysen geeignet.** Zur Auswertung werden das mindestens eine aufgenommene Spektrum der Flüssigkeit und das mindestens eine Referenzspektrum bzw. werden die zugehörigen Messdaten geeigneten Vergleichs- und/oder Rechenoperationen und/oder geeigneten Transformationen unterzogen wie sie einem Fachmann bekannt und geläufig sind, so dass mittels einer vergleichenden Analyse eine Quantifizierung des in der Flüssigkeit enthaltenen Polyphenols möglich ist.

Das mindestens eine Referenzspektrum wird vorzugsweise vor der Durchführung des Verfahrens aufgenommen, und zwar vorzugsweise unter der Verwendung der derselben Messvorrichtung, welche auch für das Verfahren selbst verwendet wird. Alternativ kann das mindestens eine Referenzspektrum auch während des Verfahrens aufgenommen werden, wobei lediglich darauf zu achten ist, dass das wenigstens eine Referenzspektrum vor Schritt c) des Verfahrens aufgenommen wird. Auf diese Art und Weise kann beispielsweise sichergestellt werden, dass zu jedem Spektrum einer zu stabilisierenden Flüssigkeit ein aktuelles Referenzspektrum vorliegt. Alternativ können aber auch Referenzspektren für jeweils zu stabilisierende Flüssigkeiten bereitgestellt werden, welche unabhängig von der Durchführung oder der Vorrichtung des Verfahrens bereits zu einem früheren Zeitpunkt aufgenommen und gespeichert wurden. Beispielsweise kann je nach Anwendungsfall abhängig von bestimmten Vorgaben und Betriebsregeln oder nach Ermessen eines Fachmannes ein aufgenommenes Referenzspektrum über einen vorgegebenen Zeitraum verwendet werden, bevor nach Ablauf des vorgegebenen Zeitraums erneut ein Referenzspektrum zur weiteren Verwendung aufgenommen wird.

Wie bekannt, besteht die Gruppe der Polyphenole aus einer größeren Anzahl von Polyphenolen, welche sich hinsichtlich bestimmter Eigenschaften voneinander unterscheiden. Um diesen Umstand zu berücksichtigen, wird gemäß einer bevorzugten Ausführungsform des vorliegenden Verfahrens wenigstens ein Referenzspektrum von zumindest einer Art eines Polyphenols aufgenommen oder bereitgestellt, wobei das mindestens eine Spektrum der zu stabilisierenden Flüssigkeit mit mindestens einem der Referenzspektren verglichen wird und aus den Vergleichsdaten der Wert für die quantitative Menge der in der Flüssigkeit enthaltenen zumindest einen Art von Polyphenol errechnet wird.

Für das Referenzspektrum kann beispielsweise eine Art von Polyphenol herangezogen werden, welche basierend auf empirischen und/oder wissenschaftlichen Grundlagen auch in der zu stabilisierenden Flüssigkeit zu erwarten ist. Dadurch ist eine präzisere Auswertung möglich, da das Spektrum der Flüssigkeit und das mindestens eine Referenzspektrum vorzugsweise in einem definierten, eingeschränkten Spektralbereich, in dem ein für die verwendete Art von Polyphenol charakteristisches Spektrallinienmuster vorhanden ist, genau verglichen werden können.

Insbesondere bevorzugt werden während des laufenden Verfahrens, also während der Änderung der Konzentration an Polyphenol mehrere Spektren aufgenommen, welche dann in Art einer Kalibrierungsreihe oder Eichreihe für die vergleichende Auswertung und Analyse zur Verfügung stehen. Beispielsweise kann das Spektrum der Flüssigkeit bzw. können die anhand der Rechenoperationen und/oder Transformationen erhaltenen Daten des Spektrums der Flüssigkeit mit den konzentrationsabhängig erhaltenen Daten aus den Referenzspektren verglichen werden. Durch diesen Abgleich kann aus dem Spektrum der Flüssigkeit anhand der als Kalibrierungs- oder Eichreihe aufbereiteten Daten der Referenzspektren ein Mengen- oder Konzentrationswert an Polyphenol in der Flüssigkeit abgelesen werden.

Die zumindest eine Art von Polyphenol, von welcher ein Referenzspektrum aufgenommen oder bereitgestellt wird und welche somit für die vergleichende Analyse herangezogen wird, wird beispielsweise aufgrund empirischer Daten bzw. mit Hilfe von Erfahrungswerten aus der Praxis ausgewählt. Dabei wird vorzugsweise eine bestimmte Art von Polyphenol gewählt, welche, gemäß der häufig wissenschaftlich belegten, praktischen Erfahrungswerte, auch in der zu stabilisierenden Flüssigkeit enthalten ist. Solche Arten von Polyphenolen, die in einer bestimmten Flüssigkeit zu erwarten sind bzw. immer in der zu stabilisierenden Flüssigkeit vorhanden sind, und/oder den größten Mengenanteil an Polyphenol ausmachen, werden als Leit-Polyphenole für diese Flüssigkeit bezeichnet. Aus vergleichenden Analysen des Spektrums der Flüssigkeit und zumindest eines Referenzspektrums eines Leit-Polyphenols kann beispielsweise die in der Flüssigkeit enthaltene, quantitative Menge des Leit-Polyphenols ermittelt werden.

Gemäß einer bevorzugten Ausführungsform der vorliegenden Erfindung wird eine zumindest zwei Arten von Polyphenol oder zumindest drei Arten von Polyphenol oder eine Vielzahl an Arten von Polyphenol enthaltende Flüssigkeit bereitgestellt, wobei die quantitative Menge des insgesamt in der Flüssigkeit enthaltenen Polyphenols ermittelt wird. Insbesondere bevorzugt wird dabei zunächst ein Wert für die quantitative Menge eines Leit-Polyphenols ermittelt und aus dem erhaltenen Wert für die quantitative Menge eines Leit-Polyphenols wird anschließend mittels geeigneter Auswerteoperationen die quantitative Menge der insgesamt in der Flüssigkeit enthaltenen Polyphenole errechnet. Vorteilhaft kann bei zu stabilisierenden Flüssigkeiten, die zumindest zwei, zumindest drei oder eine Vielzahl an Arten von Polyphenol enthalten, eine geeignete Auswahl zumindest einer Art von Leit-Polyphenol, welches für die vergleichende Analyse herangezogen wird, getroffen werden. Die zumindest eine Art von Leit-Polyphenol wird, wie bereits beschrieben, aufgrund empirischer Daten bzw. mit Hilfe von Erfahrungswerten aus der Praxis ausgewählt und die quantitative Menge des Leit-Polyphenols wird zunächst in der zu stabilisierenden Flüssigkeit ermittelt. Der so erhaltene Mengenwert für die quantitative Menge des Leit-Polyphenols kann nunmehr umgerechnet werden auf einen Mengenwert für das insgesamt in der Flüssigkeit enthaltene Polyphenol. Diese Umrechnung basiert auf weiteren empirischen und wissenschaftlichen Daten bzw. auf Erfahrungswerten aus der Praxis. Zwar fehlen in der Praxis jegliche Hinweise und Daten über die in einer zu stabilisierenden Flüssigkeit enthaltene Absolutmenge an Polyphenolen bzw. über die Konzentration der insgesamt enthaltenen Polyphenole in einer zu stabilisierenden Flüssigkeit, jedoch sind häufig die Arten von enthaltenen Polyphenolen sowie die ungefähre anteilsmäßige Verteilung oder die ungefähren Verhältnisse bestimmter Arten von Polyphenolen zueinander in einer vorgegebenen Flüssigkeit bekannt. Mittels dieser bekannten Daten kann somit aus dem ermittelten Mengenwert des Leit-Polyphenols auf einen Wert für die Menge von insgesamt enthaltenen Polyphenolen geschlossen werden.

Ebenso bevorzugt werden zum Ermitteln der quantitativen Menge des insgesamt in der Flüssigkeit enthaltenen Polyphenols zunächst Werte für die quantitative Menge von wenigstens zwei, drei oder mehreren Arten von Leit-Polyphenolen ermittelt. Vorzugsweise werden dazu Referenzspektren von wenigstens zwei, drei oder mehreren Arten von Leit-Polyphenolen aufgenommen oder bereitgestellt, welche dann für eine vergleichende Auswertung zur Verfügung stehen. Aus dem Vergleich wird der Mengenwert für die quantitative Menge des insgesamt in der Flüssigkeit enthaltenen Polyphenols errechnet und zwar basierend auf den bereits erläuterten Erfahrungswerten bezüglich der anteilsmäßigen Verteilung von Polyphenolen in bestimmten Flüssigkeiten.

Besonders bevorzugt wird das Verfahren zur Stabilisierung eines polyphenolhaltigen Getränkes eingesetzt, wobei insbesondere bevorzugt polyphenolhaltiges Bier bereitgestellt wird. Vorzugsweise sind, insbesondere wenn Bier als die zu stabilisierende Flüssigkeit verwendet wird, die zumindest eine Art oder die zwei, oder drei oder mehreren Arten von Leit-Polyphenol ausgewählt aus einer Gruppe von Flavonoiden, bevorzugt aus einer Gruppe von Chalkonen und/oder Flavonen und/oder Anthocyanidinen, besonders bevorzugt aus einer Gruppe von Proanthocyanidinen und insbesondere bevorzugt aus einer Gruppe von dimeren B-Typ-Proanthocyanidinen. Ganz besonders bevorzugt handelt es sich bei der zumindest einen Art oder den zwei, oder drei oder mehreren Arten von Leit-Polyphenol um Procyanidin B3 oder Flavan-3-ol-Dimer oder Prodelphinidin B3 oder Xanthohumol oder um ein beliebiges Gemisch aus Procyanidin B3 und/oder Flavan-3-ol-Dimer und/oder Prodelphinidin B3 und/oder Xanthohumol.

Der in Bier enthaltene Anteil an Polyphenolen stammt aus dem Malz und aus dem verwendeten Hopfen, wobei es vor allem unter den so genannten Hopfenpolyphenolen ganz spezifische Arten gibt, die fast ausschließlich in Hopfen vorkommen. Bei den oben genannten Arten von Polyphenolen handelt es sich beispielsweise um solche Hopfenpolyphenole, welche durch den Eintrag des Hopfens beim Bierbrauen als Leit-Polyphenole in Bier angesehen werden können. Insbesondere bevorzugt werden diese Arten an Polyphenolen deshalb bei der Durchführung des Verfahrens zur Stabilisierung von Bier als Leit-Polyphenole ausgewählt.

Das Verfahren wird in vorgegebenen Zeitabständen wiederholt. Vorliegend ist darunter zu verstehen, dass das Verfahren als kontinuierliches Verfahren durchgeführt wird, welches in einem laufenden Prozess, der Getränkeherstellung und -abfüllung integriert durchgeführt wird. Sämtliche Verfahrensschritte werden dabei aufeinanderfolgend in vorgegebenen Zeitabständen wiederholt, so dass die Stabilisierung als so genannter Inline-Prozess durchgeführt wird. Die Ermittlung des Wertes der quantitativen Menge an Polyphenol in der zu stabilisierenden Flüssigkeit ist somit auch als analytischer Schritt eines Inline-Prozesses zu verstehen der wiederkehrend während des laufenden Gesamtprozesses wiederholt wird.

Beispielsweise wird zu stabilisierendes Bier, aus einer Maisch- oder Gär- oder Vorfiltrationseinheit kommend in einem Produktstrom in die Bereitstellungs- und Zuführeinheit eingeleitet und bereitgestellt. Von dort aus durchströmt das Bier die Messvorrichtung, in welcher beispielsweise mittels IR-Spektroskopie das IR-Spektrum des Bieres und gegebenenfalls das IR-Referenzspektrum aufgenommen werden oder wurden. An der mit der Messvorrichtung verschalteten Vergleichs- und Auswerteeinheit laufen im Wesentlichen parallel die Analysen und Berechnungen zur Ermittlung des Mengenwertes an Polyphenol im Bier. Nach Durchlaufen der Messvorrichtung passiert der Produktstrom die Dosiereinheit, mit Hilfe derer das Stabilisierungsmittel mengenproportional in Abhängigkeit des ermittelten Mengenwertes an Polyphenol in den Produktstrom zugegeben wird. Nach der Zugabe des Stabilisierungsmittels wird das Gemisch aus Bier und Stabilisierungsmittel einer Filtereinheit zur Abtrennung des Stabilisierungsmittels zugeführt und schließlich wird das stabilisierte Bier mittels der Abführleitung abgeführt und beispielsweise einem Pufferbehälter oder Tank oder einer Abfüllanlage zugeführt. Sämtliche Schritte werden in vorgegebenen zeitlichen Abständen wiederholt durchgeführt, so dass über eine gesamte Produktionscharge des Bieres hinweg eine in Grad und Qualität gleichbleibende Stabilisierung erfolgt.

Als Verfahren zur Durchführung der Spektroskopie sind alle Verfahren geeignet, die es erlauben, die untersuchte Flüssigkeit zur Ausbildung eines analysierbaren Spektrums anzuregen. Dazu sind insbesondere, aber nicht beschränkend solche Verfahren geeignet, welche die zu untersuchende Flüssigkeit mit sichtbarem Licht, unsichtbarem Licht, Infrarotstrahlung, Near-Infrared-Strahlung, Laser oder elektromagnetischer Strahlung mit geeigneter Wellenlänge beaufschlagen.

Als Stabilisierungsmittel wird insbesondere bevorzugt Polyvinylpolypyrrolidon (PVPP) verwendet. Alternativ können jedoch auch andere Stabilisierungsmittel, beispielsweise in der Lebensmitteltechnologie als unbedenklich einsetzbare Absorber verwendet werden. Ebenfalls können auch Enzyme eingesetzt werden. Ebenso ist es denkbar, neben der Ermittlung der Menge an Polyphenolen in Flüssigkeiten mit dem vorliegenden Verfahren auch andere bzw. weitere Stör- oder Schadstoffe sowohl qualitativ als auch quantitativ zu bestimmen, sofern geeignete Referenz-Substanzen sowie geeignete Messbedingungen und Vergleichs- und Berechnungsoperationen vorliegen und berücksichtigt werden können.

Gemäß einer bevorzugten Ausführungsform der vorliegenden Erfindung wird zum Ermitteln der quantitativen Menge des insgesamt in der Flüssigkeit enthaltenen Polyphenols eine Infrarot-Spektroskopie im mittleren Infrarot, vorzugsweise in einem Wellenzahlbereich von 4000 cm⁻¹ bis 400 cm⁻¹ oder im nahen Infrarot, vorzugsweise in einem Wellenzahlbereich von 12500 cm⁻¹ bis 4000 cm⁻¹ durchgeführt. Insbesondere bevorzugt wird zum Ermitteln der quantitativen Menge des insgesamt in der Flüssigkeit enthaltenen Polyphenols eine IR-spektroskopische Messung in einem vorbestimmten Wellenzahlbereich durchgeführt, wobei der Wellenzahlbereich maximal 50 cm⁻¹ umfasst.

Die vorliegende Erfindung umfasst auch eine Vorrichtung zum Durchführen des Verfahrens zur Stabilisierung einer zumindest eine Art von Polyphenol enthaltenden Flüssigkeit gemäß der Erfindung. Die Vorrichtung weist wenigstens
eine Bereitstellungs- und Zuführeinheit zum Bereitstellen der zumindest eine Art von Polyphenol enthaltenden Flüssigkeit,
wenigstens eine Messvorrichtung zur Durchführung einer Spektroskopie, wobei die Messvorrichtung zumindest zur Aufnahme wenigstens eines Spektrums der Flüssigkeit ausgebildet ist,
eine Vergleichs- und Auswerteeinheit für den Vergleich von Spektren und der Berechnung eines sich aus dem Vergleich durch geeignete Vergleichsoperationen ergebenden Mengenwertes für die quantitative Menge des in der Flüssigkeit enthaltenen Polyphenols,
eine Dosiereinheit zum Zusetzen einer bestimmten Menge eines in der Flüssigkeit unlöslichen Stabilisierungsmittels zu der Flüssigkeit,
eine Filtereinheit zum Abtrennen des wenigstens teilweise mit Polyphenol beladenen Stabilisierungsmittels und
eine Abführleitung zum Abführen der von der wenigstens einen Art von Polyphenol abgereicherten, stabilisierten Flüssigkeit auf.

Vorteilhaft steht die Messvorrichtung zur Durchführung der Spektroskopie mit der Vergleichs- und Auswerteeinheit in kommunizierender Verbindung. Die Vergleichs- und Auswerteeinheit verfügt vorzugsweise über geeignete Mittel zur Aufnahme, Verrechnung, Ausgabe und Speicherung der durch die Messvorrichtung erzeugten Rohdaten, beispielsweise umfasst die Vergleichs- und Auswerteeinheit Mittel zur elektronischen Bildverarbeitung.

Besonders vorteilhaft verfügt die Vorrichtung zusätzlich über eine weitere Messvorrichtung und insbesondere vorteilhaft ist zusätzlich eine Steuereinheit vorgesehen, wobei die Steuereinheit zur kommunizierenden Verbindung mit der Vergleichs- und Auswerteeinheit und zur Steuerung der Dosiereinheit ausgebildet ist. Besonders bevorzugt ist die Vorrichtung für den Einsatz bzw. für die Integration in einer Anlage zur Getränkeherstellung ausgebildet.

### Kurze Beschreibung der Zeichnungen

Die Erfindung soll nachfolgend anhand von Ausführungsbeispielen im Zusammenhang mit den Zeichnungen näher erläutert werden. Es zeigen
- Fig. 1: ein Übersichtsdiagramm einer Vorrichtung zur Durchführung des erfindungsgemäßen Verfahrens und
- Fig. 2: ein Übersichtsdiagramm einer Vorrichtung zur Durchführung einer weiteren Ausführungsvariante des vorliegenden Verfahrens.

### Wege zur Ausführung der Erfindung

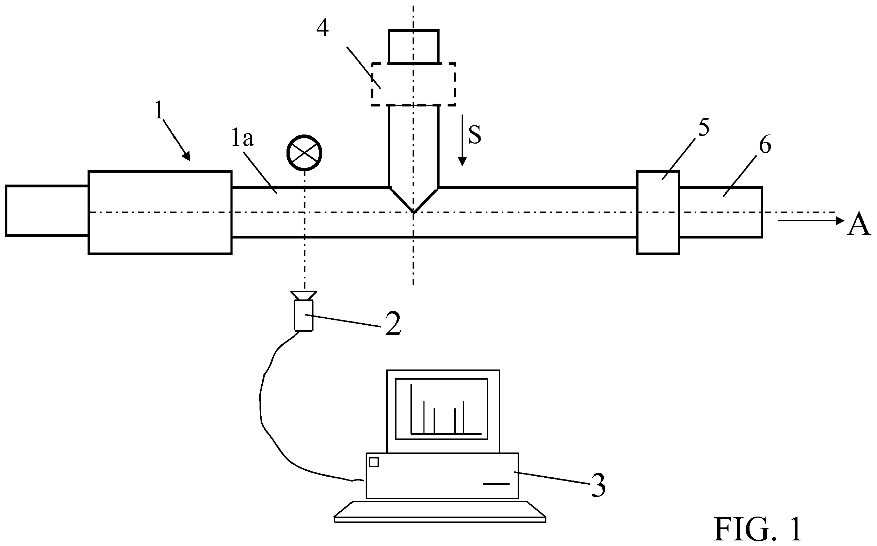

Fig. 1 zeigt ein Übersichtsdiagramm einer Vorrichtung zur Durchführung des erfindungsgemäßen Verfahrens zur Stabilisierung einer Flüssigkeit, wobei im dargestellten Beispiel Bier stabilisiert wird. Das Verfahren wird als kontinuierliches Verfahren durchgeführt, wobei die Verfahrensschritte in kontinuierlicher Weise ablaufen und in den Gesamtprozess der Herstellung und Abfüllung des Bieres integriert sind. Das Bier wird aus einer Maisch-, Gär- oder Vorfiltrationseinheit kommend in eine Bereitstellungs- und Zuführeinheit 1 einer Vorrichtung zur Durchführung des Verfahrens eingeleitet und bereitgestellt. Das Bier durchströmt in einem Produktstrom A die Vorrichtung, wobei es zunächst zu einer in einem lichtdurchlässigen Abschnitt 1a der Bereitstellungs- und Zuführeinheit 1 angeordneten Messvorrichtung 2 geleitet wird.

Bei der Messvorrichtung 2 handelt es sich um ein Spektrometer, im Beispiel um ein Infrarot-Spektrometer (IR). Mit Hilfe der Messvorrichtung 2 wird ein IR-Spektrum des Bieres und gegebenenfalls ein oder mehrere IR-Referenzspektren einer oder mehrerer Arten von Leit-Polyphenolen aufgenommen. Zur Messung des IR-Spektrums des Bieres im Produktstrom A wird in dem lichtdurchlässigen Abschnitt 1a der Bereitstellungs- und Zuführeinheit 1 Infrarotlicht mit einer bestimmten Wellenzahl, im dargestellten Beispiel im mittleren Infrarot, in einem Wellenzahlbereich von 4000 cm⁻¹ bis 400 cm⁻¹, in den Produktstrom A eingestrahlt. Das Bier im Produktstrom A absorbiert einen Teil des eingestrahlten Lichts. Das Restlicht wird über die Messvorrichtung 2 detektiert und mittels einer mit der Messvorrichtung 2 verbundenen Vergleichs- und Auswerteeinheit 3 auf das Vorhandensein eines Spektrallinienmusters untersucht.

Mit der Vergleichs- und Auswerteeinheit 3 werden im Wesentlichen gleichzeitig Analysen und Berechnungen zur Ermittlung des Mengenwertes an Polyphenol im Bier durchgeführt, indem die erhaltenen Messdaten des IR-Spektrums des Bieres verglichen werden mit den Daten des IR-Referenzspektrums und gegebenenfalls mit einem IR-Vergleichsspektrum oder mit mehreren IR-Referenzspektren einer oder mehrerer Arten von Leit-Polyphenolen. Dazu werden in der Vergleichs- und Auswerteeinheit 3 geeignete, dem Fachmann bekannte Vergleichs- und/oder Rechenoperationen und/oder Transformationen durchgeführt, so dass mittels einer vergleichenden Analyse eine Quantifizierung des im Bier enthaltenen Polyphenols erzielt wird.

Nach Durchlaufen der Messvorrichtung 2 passiert der Produktstrom A eine Dosiereinheit 4, welche durch eine mit der Vergleichs- und Auswerteeinheit 3 in kommunizierender Verbindung stehenden Steuereinheit (nicht im Diagramm dargestellt) geregelt wird. Mit Hilfe der über die Steuereinheit geregelten Dosiereinheit 4 wird das Stabilisierungsmittel in einem Zuführstrom S in den Produktstrom A zugegeben. Zur Zuführung des Stabilisierungsmittels, im dargestellten Beispiel Polyvinylpolypyrrolidon (PVPP), wird der Zuführstrom S dabei in Abhängigkeit des ermittelten Mengenwertes an Polyphenol im Bier geregelt, so dass das Stabilisierungsmittel mengenproportional, eingestellt auf den Polyphenolgehalt des Bieres, in den Produktstrom zugegeben wird.

Nach der Zugabe des Stabilisierungsmittels wird das Gemisch aus Bier und Stabilisierungsmittel in der Produktstromrichtung weitergeleitet und einer Filtereinheit 5 zugeführt. Die Verweildauer, in der das Bier mit dem Stabilisierungsmittel im Produktstrom A in Kontakt ist, kann beispielsweise über die Strömungsgeschwindigkeit des Produktstromes A oder über die räumliche Distanz zwischen dem Ort der Einleitung des Zuführstromes S in den Produktstrom A und der Filtereinheit 5, eingestellt werden. An der Filtereinheit 5 erfolgt die Abtrennung des Stabilisierungsmittels vom Bier gemäß aus dem Stand der Technik bekannten Filtrationsmethoden. Das stabilisierte Bier kann schließlich mittels einer Abführleitung 6 abgeführt und beispielsweise zu einem Pufferbehälter oder Tank oder einer Abfüllanlage geleitet werden.

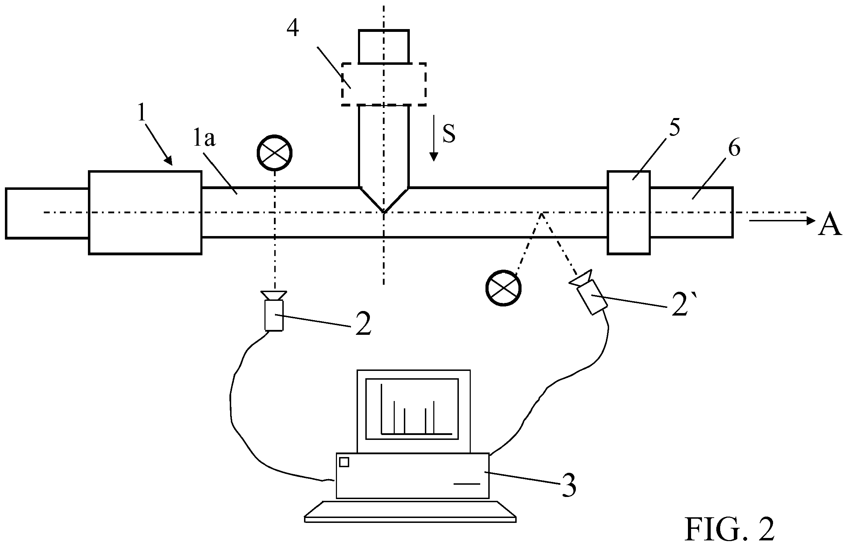

In der Figur 2 ist ein Übersichtsdiagramm einer Vorrichtung zur Durchführung einer weiteren Ausführungsvariante des vorliegenden Verfahrens zur Stabilisierung von Bier dargestellt. In dem vorliegenden Beispiel der Figur 2 wird mittels einer zusätzlich vorhandenen, weiteren Messvorrichtung 2'ein weiteres IR-Spektrum des Bieres im Produktstrom A aufgenommen. Bezogen auf die Richtung des Produktstroms A ist die weitere Messvorrichtung 2' der Messvorrichtung 2 sowie der Dosiereinheit 4 nachgeschaltet. Im dargestellten Beispiel wird mit der Messvorrichtung 2 ein erstes IR-Spektrum des Bieres aufgenommen und in Abhängigkeit des über die Vergleichs- und Auswerteeinheit 3 ermittelten Mengenwertes an Polyphenol im Bier wird über die Dosiereinheit 4 das Stabilisierungsmittel mengenproportional zugesetzt. Nach dem Zuführen des Stabilisierungsmittels wird mittels der weiteren Messvorrichtung 2'ein zweites IR-Spektrum des Bieres aufgenommen.

Zur Messung des zweiten IR-Spektrums des Bieres im Produktstrom A wird wiederum Infrarotlicht mit einer bestimmten Wellenzahl, im dargestellten Beispiel wieder im mittleren Infrarot, in einem Wellenzahlbereich von 4000 cm⁻¹ bis 400 cm⁻¹, in den Produktstrom A eingestrahlt. Reflektiertes Licht wird über die weitere Messvorrichtung 2' detektiert und mittels der mit der Messvorrichtung 2' verbundenen Vergleichs- und Auswerteeinheit 3 auf das Vorhandensein eines Spektrallinienmusters untersucht.

Durch Auswertung der erhaltenen Daten aus dem zweiten IR-Spektrum des Bieres kann der Stabilisierungsgrad kontrolliert werden. Sollte der gewünschte Stabilisierungsgrad nicht erreicht sein, kann in Reaktion darauf beispielsweise die Verweildauer, in der das Bier mit dem Stabilisierungsmittel im Produktstrom A in Kontakt ist, durch Verlangsamung der Strömungsgeschwindigkeit des Produktstromes A erhöht werden. Alternativ kann über die Dosiereinheit 4 zusätzliches Stabilisierungsmittel nachdosiert werden.

### Bezugszeichenliste

- 1: Bereitstellungs- und Zuführeinheit
- 1a: lichtdurchlässiger Abschnitt
- 2, 2': Messvorrichtung
- 3: Vergleichs- und Auswerteeinheit
- 4: Dosiereinheit
- 5: Filtereinheit
- 6: Abführleitung

- A: Produktstrom
- S: Zuführstrom

## Patentansprüche

1. Verfahren zur Stabilisierung einer zumindest eine Art von Polyphenol enthaltenden Flüssigkeit, umfassend die Schritte
a) Bereitstellen einer zumindest eine Art von Polyphenol enthaltenden Flüssigkeit in einer Bereitstellungs- und Zuführeinheit (1),
b) Aufnahme eines Spektrums der Flüssigkeit mittels zumindest einer vorgesehenen und dafür eingerichteten Messvorrichtung (2),
c) Ermitteln einer quantitativen Menge der in der Flüssigkeit enthaltenen zumindest einen Art von Polyphenol mittels einer vorgesehenen und dafür eingerichteten Vergleichs- und Auswerteeinheit (3), wobei die Vergleichs- und Auswerteeinheit (3) das in Schritt b) aufgenommene Spektrum der Flüssigkeit mit wenigstens einem Referenzspektrum vergleicht und aus dem Vergleich durch geeignete Vergleichsoperationen einen Wert für die quantitative Menge der in der Flüssigkeit enthaltenen zumindest einen Art von Polyphenol errechnet,
d) Zusetzen einer bestimmten Menge eines in der Flüssigkeit unlöslichen Stabilisierungsmittels zu der Flüssigkeit mittels einer Dosiereinheit (4), wobei die Menge an Stabilisierungsmittel in Abhängigkeit des in Schritt c) errechneten Wertes für die quantitative Menge an Polyphenol bestimmt wird und wobei das Stabilisierungsmittel zum Binden von wenigstens einer Art von Polyphenol ausgebildet ist,
e) Zuführen des Gemisches aus Flüssigkeit und Stabilisierungsmittel zu zumindest eine vorgesehene Filtereinheit (5),
f) Abtrennen des wenigstens teilweise mit Polyphenol beladenen Stabilisierungsmittels mittels der Filtereinheit (5),
g) Abführen der von der wenigstens einen Art von Polyphenol abgereicherten, stabilisierten Flüssigkeit mittels einer vorgesehenen Abführleitung (6),
wobei das Verfahren als kontinuierlicher inline-Prozess in einem laufenden Prozess der Getränkeherstellung und -abfüllung durchführbar ist.

2. Verfahren nach Anspruch 1, **dadurch gekennzeichnet, dass** vor Schritt c) das Referenzspektrum mittels der Messvorrichtung (2) aufgenommen wird.

3. Verfahren nach Anspruch 1 oder 2, **dadurch gekennzeichnet, dass** vor Schritt c) wenigstens ein Referenzspektrum zumindest einer Art eines Polyphenols aufgenommen oder bereitgestellt wird und in Schritt c) die quantitative Menge der in der Flüssigkeit enthaltenen zumindest einen Art von Polyphenol mittels der vorgesehenen und dafür eingerichteten Vergleichs- und Auswerteeinheit (3) ermittelt wird, wobei die Vergleichs- und Auswerteeinheit (3) das in Schritt b) aufgenommene Spektrum der Flüssigkeit mit dem wenigstens einen Referenzspektrum vergleicht und aus dem Vergleich durch geeignete Vergleichsoperationen einen Wert für die quantitative Menge der in der Flüssigkeit enthaltenen zumindest einen Art von Polyphenol errechnet.

4. Verfahren nach einem der Ansprüche 1 bis 3, **dadurch gekennzeichnet, dass** in Schritt a) eine zumindest zwei Arten von Polyphenol oder zumindest drei Arten von Polyphenol oder eine Vielzahl an Arten von Polyphenol enthaltende Flüssigkeit bereitgestellt wird, wobei in Schritt c) die quantitative Menge der insgesamt in der Flüssigkeit enthaltenen Polyphenole ermittelt wird.

5. Verfahren nach Anspruch 4, **dadurch gekennzeichnet, dass** in Schritt c) mittels der Vergleichs- und Auswerteeinheit (3) aus dem Vergleich des Spektrums der Flüssigkeit mit dem wenigstens einen IR-Referenzspektrum durch geeignete Vergleichsoperationen zunächst ein Wert für die quantitative Menge zumindest einer Art eines Leit-Polyphenoles in der Flüssigkeit ermittelt wird und aus dem erhaltenen Wert für die quantitative Menge der zumindest einen Art des Leit-Polyphenoles mittels vorgegebener Auswerteoperationen die quantitative Menge der insgesamt in der Flüssigkeit enthaltenen Polyphenole errechnet wird.

6. Verfahren nach Anspruch 4 oder 5, **dadurch gekennzeichnet, dass** zum Ermitteln der quantitativen Menge des insgesamt in der Flüssigkeit enthaltenen Polyphenols zunächst Werte für die quantitative Menge von zwei, drei oder mehreren Arten an Leit-Polyphenolen in der Flüssigkeit ermittelt werden und aus den erhaltenen Werten für die quantitative Menge der zwei, drei oder mehreren Arten an Leit-Polyphenolen mittels vorgegebener Auswerteoperationen die quantitative Menge der insgesamt in der Flüssigkeit enthaltenen Polyphenole errechnet wird.

7. Verfahren nach einem der vorhergehenden Ansprüche, **dadurch gekennzeichnet dass** in Schritt a) ein polyphenolhaltiges Getränk bereitgestellt wird.

8. Verfahren nach einem der Ansprüche 5 bis 7, **dadurch gekennzeichnet, dass** die zumindest eine Art oder die zwei, oder drei oder mehreren Arten von Leit-Polyphenolen ausgewählt sind aus einer Gruppe von Flavonoiden, bevorzugt aus einer Gruppe von Chalkonen und/oder Flavonen und/oder Anthocyanidinen, besonders bevorzugt aus einer Gruppe von Proanthocyanidinen und insbesondere bevorzugt aus einer Gruppe von dimeren B-Typ-Proanthocyanidinen.

9. Verfahren nach Anspruch 8, **dadurch gekennzeichnet, dass** die zumindest eine Art oder die zwei, oder drei oder mehreren Arten von Leit-Polyphenolen Procyanidin B3 oder Flavan-3-ol-Dimer oder Prodelphinidin B3 oder Xanthohumol oder ein beliebiges Gemisch aus Procyanidin B3 und/oder Flavan-3-ol-Dimer und/oder Prodelphinidin B3 und/oder Xanthohumol sind.

10. Verfahren nach einem der vorhergehenden Ansprüche, **dadurch gekennzeichnet, dass** das Verfahren in vorgegebenen Zeitabständen wiederholt wird.

11. Verfahren nach einem der vorhergehenden Ansprüche, **dadurch gekennzeichnet, dass** als Stabilisierungsmittel Polyvinylpolypyrrolidon (PVPP) verwendet wird.

12. Verfahren nach einem der vorhergehenden Ansprüche, **dadurch gekennzeichnet, dass** in Schritt b) ein IR-Spektrum der Flüssigkeit im mittleren Infrarot, vorzugsweise in einem Wellenzahlbereich von 4000 cm⁻¹ bis 400 cm⁻¹ oder im nahen Infrarot, vorzugsweise in einem Wellenzahlbereich von 12500 cm⁻¹ bis 4000 cm⁻¹ durchgeführt wird.

13. Verfahren nach einem der vorhergehenden Ansprüche, **dadurch gekennzeichnet, dass** in Schritt b) eine IR-spektroskopische Messung in einem vorbestimmten Wellenzahlbereich durchgeführt wird, wobei der Wellenzahlbereich maximal 50 cm⁻¹ umfasst.

14. Verfahren nach einem der vorhergehenden Ansprüche, **dadurch gekennzeichnet, dass** es sich bei der Spektroskopie um eine Near-Infrared-Spektroskopie und/oder eine Spektroskopie mit sichtbarem Licht und/oder eine Spektroskopie mit unsichtbarem Licht und/oder Laserspektroskopie und/oder elektromagnetischer Strahlung mit geeigneter Wellenlänge handelt.

15. Vorrichtung zum Durchführen eines Verfahrens zur Stabilisierung einer zumindest eine Art von Polyphenol enthaltenden Flüssigkeit gemäß einem der Ansprüche 1 bis 14, wenigstens aufweisend
- eine Bereitstellungs- und Zuführeinheit (1) zum Bereitstellen der zumindest eine Art von Polyphenol enthaltenden Flüssigkeit,
- eine Messvorrichtung (2) zur Durchführung einer Spektroskopie, wobei die Messvorrichtung (2) zur Aufnahme wenigstens eines Spektrums der Flüssigkeit ausgebildet ist,
- eine Vergleichs- und Auswerteeinheit (3) für den Vergleich von Spektren und der Berechnung eines sich aus dem Vergleich durch geeignete Vergleichsoperationen ergebenden Wertes für die quantitative Menge des in der Flüssigkeit enthaltenen Polyphenols,
- eine Dosiereinheit (4) zum Zusetzen einer bestimmten Menge eines in der Flüssigkeit unlöslichen Stabilisierungsmittels zu der Flüssigkeit,
- eine Filtereinheit (5) zum Abtrennen des wenigstens teilweise mit Polyphenol beladenen Stabilisierungsmittels und
- eine Abführleitung (6) zum Abführen der von der wenigstens einen Art von Polyphenol abgereicherten, stabilisierten Flüssigkeit,
wobei die Vorrichtung derart ausgebildet ist, dass das Verfahren der Ansprüche 1 bis 14 als kontinuierlicher inline-Prozess in einem laufenden Prozess der Getränkeherstellung und -abfüllung durchführbar ist.

16. Vorrichtung nach Anspruch 15, **dadurch gekennzeichnet, dass** zusätzlich eine Steuereinheit vorgesehen ist, wobei die Steuereinheit zur kommunizierenden Verbindung mit der Vergleichs- und Auswerteeinheit (3) und zur Steuerung der Dosiereinheit (4) ausgebildet ist.

17. Vorrichtung nach Anspruch 15 oder 16, **dadurch gekennzeichnet, dass** zusätzlich eine weitere Messvorrichtung (2') vorgesehen ist.

## Claims

1. Method for stabilising a liquid containing at least one type of polyphenol, comprising the steps:
a) providing a liquid containing at least one type of polyphenol in a provision and delivery unit (1),
b) determining and recording a spectrum of the liquid by means of at least one measuring device (2), provided and configured for this purpose,
c) determining a quantitative amount of the at least one type of polyphenol contained in the liquid by means of a comparison and evaluation unit (3), provided and configured for this purpose, wherein the comparison and evaluation unit (3) compares the spectrum of the liquid recorded in step b) with at least one reference spectrum, and, from the comparison, by means of suitable comparison operations, calculates a value for the quantitative amount of the at least one type of polyphenol contained in the liquid,
d) adding a specific quantity of a stabilising medium, insoluble in the liquid, to the liquid by means of a metering unit (4), wherein the amount of the stabilising medium is determined as a dependency of the value calculated in step c) for the quantitative amount of polyphenol, and wherein the stabilising medium is configured such as to bond with at least one type of polyphenol,
e) adding the mixture of liquid and stabilising medium to at least one filter unit (5) provided,
f) separating off the stabilising medium at least partially laden with polyphenol by means of the filter unit (5),
g) drawing off the stabilised liquid enriched by the at least one type of polyphenol by means of a draw-off line (6) provided for this purpose,
wherein the method can be carried out as a continuous inline process in an ongoing process of beverage manufacture and filling.

2. Method according to claim 1, **characterised in that**, before step c), the reference spectrum is determined and recorded by means of the measuring device (2).

3. Method according to claim 1 or 2, **characterised in that**, before step c), at least one reference spectrum of at least one type of polyphenol is recorded or provided, and in step c) the quantitative amount of the at least one type of polyphenol contained in the liquid is determined by means of the comparison and evaluation unit (3), provided and configured for this purpose, wherein the comparison and evaluation unit (3) compares the spectrum of the liquid recorded in step b) with the at least one reference spectrum, and, from the comparison, by means of suitable comparison operations, calculates a value for the quantitative amount of the at least one type of polyphenol contained in the liquid.

4. Method according to any one of claims 1 to 3, **characterised in that** in step a) a liquid is provided which contains at least two types of polyphenol or at least three types of polyphenol or a plurality of types of polyphenol, wherein, in step c), the overall quantitative amount of the polyphenols contained in the liquid is determined.

5. Method according to claim 4, **characterised in that**, in step c), by means of the comparison and evaluation unit (3), from the comparison of the spectrum of the liquid with the at least one IR reference spectrum by means of suitable comparison operations, first a value is determined for the quantitative amount of at least one type of a conducting polyphenol in the liquid is determined, and, from the value obtained for the quantitative amount of the at least one type of conducting polyphenol, by means of specified evaluation operations, the quantitative amount of the total of the polyphenols contained in liquid is calculated.

6. Method according to claim 4 or 5, **characterised in that**, in order to determine the quantitative amount of the total polyphenol contained in the liquid, values are first determined for the quantitative amount of two, three, or more types of conducting polyphenols in the liquid, and, from the values obtained for the quantitative amount of the two, three, or more types of conducting polyphenols, by means of specified evaluation operations, the quantitative amount of the total of the polyphenols contained in liquid is calculated.

7. Method according to any one of the preceding claims, **characterised in that** in step a) a beverage containing polyphenols is produced.

8. Method according to any one of claims 5 to 7, **characterised in that** the at least one type or the two, three, or more types of conducting polyphenols is selected from a group of flavonoids, preferably from a group of chalcones and/or flavons and/or anthocyanidins, for particular preference from a group of proanthocyanidins, and for very particular preference from a group of dimer B-type proanthocyanidins.

9. Method according to claim 8, **characterised in that** the at least one type or the two, three, or more types of conducting polyphenols is/are procyanidin B3 or flavan-3-ol-dimer, or prodelphinidin B3 or xanthohumol, or any desired mixture of procyanidin B3 and/or flavan-3-ol-dimer, and/or prodelphinidin B3 and/or xanthohumol.

10. Method according to any one of the preceding claims, **characterised in that** the method is repeated at predetermined intervals of time.

11. Method according to any one of the preceding claims, **characterised in that** use is made of polyvinyl polypyrrolidon (PVPP) as the stabilising medium.

12. Method according to any one of the preceding claims, **characterised in that**, in step b), an IR spectrum of the liquid is produced in the mid-infrared, preferably in a wavelength range from 4000 cm⁻¹ to 400 cm⁻¹ or in the near-infrared, preferably in a wavelength range from 12500 cm⁻¹ to 4000 cm⁻¹.

13. Method according to any one of the preceding claims, **characterised in that**, in step b), an IR spectroscopic measurement is carried out in a predetermined wavelength range, wherein the wavelength range comprises a maximum of 50 cm⁻¹.

14. Method according to any one of the preceding claims, **characterised in that** the spectroscopy is a near-infrared spectroscopy and/or a spectroscopy with visible light and/or a spectroscopy with invisible light and/or laser spectroscopy and/or electromagnetic radiation with suitable wavelength.

15. Device for carrying out a method for stabilising a liquid containing at least one type of polyphenol in accordance with any one of claims 1 to 14, comprising at least:
- a provision and delivery unit (1) for providing the liquid containing the at least one type of polyphenol,
- a measuring device (2) for carrying out a spectroscopy, wherein the measuring device (2) is configured such as to detect and record at least one spectrum of the liquid,
- a comparison and evaluation unit (3) for the comparison of spectra and the calculation of a value derived from the comparison by suitable comparison operations for the quantitative amount of the polyphenol contained in the liquid,
- a metering unit (4) for adding to the liquid a specific amount of a stabilising medium which is insoluble in the liquid,
- a filter unit (5) for separating off the stabilising medium at least partially laden with polyphenol, and
- a draw-off line (6) for drawing off the stabilised liquid enriched by the at least one type of polyphenol,
wherein the device is configured in such a way that the method according to claims 1 to 14 can be carried out as a continuous inline process in an ongoing process of beverage manufacture and filling.

16. Device according to claim 15, **characterised in that** a control unit is additionally provided, wherein the control unit is configured such as to provide a communicating connection to the comparison and evaluation unit (3) and to control the metering unit (4).

17. Device according to claim 15 or 16, **characterised in that** a further measuring device (2') is additionally provided.

## Revendications

1. Procédé de stabilisation d'un liquide contenant au moins un type de polyphénol, comprenant les étapes
a) de préparation d'un liquide contenant au moins un type de polyphénol dans une unité de préparation et d'amenée (1),
b) de relevé d'un spectre du liquide au moyen d'au moins un dispositif de mesure (2) prévu et mis au point à cet effet,
c) de détermination d'une quantité quantitative de l'au moins un type de polyphénol contenu dans le liquide au moyen d'une unité de comparaison et d'évaluation (3) prévue et mise au point à cet effet, dans lequel l'unité de comparaison et d'évaluation (3) compare le spectre relevé à l'étape b) du liquide à au moins un spectre de référence et calcul sur la base de la comparaison, par des opérations de comparaison adaptées, une valeur pour la quantité quantitative de l'au moins un type de polyphénol contenu dans le liquide,
d) d'ajout d'une quantité définie d'un agent stabilisant non soluble dans le liquide au liquide au moyen d'une unité de dosage (4), dans lequel la quantité en agent stabilisant est définie en fonction de la valeur calculée lors de l'étape c) pour la quantité quantitative de polyphénol et dans lequel l'agent stabilisant est réalisé pour lier au moins un type de polyphénol,
e) d'amenée du mélange composé du liquide et de l'agent stabilisant à au moins une unité de filtration (5) prévue,
f) de séparation de l'au moins un agent stabilisant chargé au moins en partie de polyphénol au moyen de l'unité de filtration (5),
g) d'évacuation du liquide stabilisé appauvri en l'au moins un type de polyphénol au moyen d'une conduite d'évacuation (6) prévue,
dans lequel le procédé peut être effectué en tant que processus en ligne continu dans un processus en cours de la fabrication et du transvasement de boissons.

2. Procédé selon la revendication 1, **caractérisé en ce qu'**avant l'étape c), le spectre de référence est relevé au moyen du dispositif de mesure (2).

3. Procédé selon la revendication 1 ou 2, **caractérisé en ce qu'**avant l'étape c), au moins un spectre de référence d'au moins un type d'un polyphénol est relevé ou préparé et à l'étape c), la quantité quantitative de l'au moins un type de polyphénol contenu dans le liquide est déterminée au moyen de l'unité de comparaison et d'évaluation (3) prévue et mise au point à cet effet, dans lequel l'unité de comparaison et d'évaluation (3) compare le spectre relevé à l'étape b) du liquide à l'au moins un spectre de référence et calcule, à partir de la comparaison, par des opérations de comparaison adaptées, une valeur pour la quantité quantitative de l'au moins un type de polyphénol contenu dans le liquide.

4. Procédé selon l'une quelconque des revendications 1 à 3, **caractérisé en ce qu'**à l'étape a) est préparé un liquide contenant au moins deux types de polyphénol ou au moins trois types de polyphénol ou une pluralité de types de polyphénol, dans lequel à l'étape c), la quantité quantitative des polyphénols contenus au total dans le liquide est déterminée.

5. Procédé selon la revendication 4, **caractérisé en ce qu'**à l'étape c), d'abord une valeur pour la quantité quantitative d'au moins un type d'un polyphénol de tête dans le liquide est déterminée au moyen de l'unité de comparaison et d'évaluation (3) à partir de la comparaison du spectre du liquide à l'au moins un spectre de référence IR par des opérations de comparaison adaptées et la quantité quantitative des polyphénols contenus au total dans le liquide est calculée à partir de la valeur obtenue pour la quantité quantitative de l'au moins un type du polyphénol de tête au moyen d'opérations d'évaluation spécifiées.

6. Procédé selon la revendication 4 ou 5, **caractérisé en ce qu'**en premier lieu des valeurs pour la quantité quantitative de deux, de trois ou de plusieurs types de polyphénols de tête dans le liquide sont déterminées pour déterminer la quantité quantitative du polyphénol contenu au total dans le liquide et la quantité quantitative des polyphénols contenus au total dans le liquide est calculée à partir des valeurs obtenues pour la quantité quantitative des deux, des trois ou des plusieurs types de polyphénols de tête au moyen d'opérations d'évaluation spécifiées.

7. Procédé selon l'une quelconque des revendications précédentes, **caractérisé en ce qu'**à l'étape a), une boisson contenant du polyphénol est préparée.

8. Procédé selon l'une quelconque des revendications 5 à 7, **caractérisé en ce que** l'au moins un type ou les deux ou les trois ou les plusieurs types de polyphénols de tête sont choisis parmi un groupe de flavonoïdes, de manière préférée parmi un groupe de chalcones et/ou de flavones et/ou d'anthocyanidines, de manière particulièrement préférée parmi un groupe de proanthocyanidines et en particulier de manière préférée parmi un groupe de proanthocyanidines dimères de type B.

9. Procédé selon la revendication 8, **caractérisé en ce que** l'au moins un type ou les deux ou trois ou les plusieurs types de polyphénols de tête sont du procyanidine B3 ou du flavan-3-ol-dimère ou du prodelphinidine B3 ou du xanthohumol ou un mélange quelconque de procyanidine B3 et/ou de flavan-3-ol-dimère et/ou du prodelphinidine B3 et/ou de xanthohumol.

10. Procédé selon l'une quelconque des revendications précédentes, **caractérisé en ce que** le procédé est répété à des intervalles spécifiés.

11. Procédé selon l'une quelconque des revendications précédentes, **caractérisé en ce que** du polyvinylpolypyrrolidone (PVPP) est utilisé en tant qu'agent stabilisant.

12. Procédé selon l'une quelconque des revendications précédentes, **caractérisé en ce qu'**à l'étape b), un spectre IR du liquide est effectué dans l'infrarouge moyen, de préférence dans une plage de nombres d'ondes de 4000 cm⁻¹ à 400 cm⁻¹ ou dans l'infrarouge proche, de préférence dans une plage de nombres d'ondes de 12 500 cm⁻¹ à 4000 cm⁻¹.

13. Procédé selon l'une quelconque des revendications précédentes, **caractérisé en ce qu'**à l'étape b), une mesure spectroscopique IR est effectuée dans une plage de nombres d'ondes prédéfinie, dans lequel la plage de nombres d'ondes est au maximum de 50 cm⁻¹.

14. Procédé selon l'une quelconque des revendications précédentes, **caractérisé en ce que** la spectroscopie est une spectroscopie dans l'infrarouge proche et/ou une spectroscopie avec de la lumière visible et/ou une spectroscopie avec de la lumière non visible et/ou une spectroscopie au laser et/ou un rayonnement électromagnétique avec une longueur d'onde adaptée.

15. Dispositif pour effectuer un procédé de stabilisation d'un liquide contenant au moins un type de polyphénol selon l'une quelconque des revendications 1 à 14, présentant au moins
- une unité de préparation et d'amenée (1) pour préparer le liquide contenant au moins un type de polyphénol,
- un dispositif de mesure (2) pour effectuer une spectroscopie, dans lequel le dispositif de mesure (2) est réalisé pour relever au moins un spectre du liquide,
- une unité de comparaison et d'évaluation (3) pour la comparaison de spectres et pour le calcul d'une valeur, résultant de la comparaison par des opérations de comparaison adaptées, pour la quantité quantitative du polyphénol contenu dans le liquide,
- une unité de dosage (4) pour ajouter au liquide une quantité définie d'un agent stabilisant non soluble dans le liquide,
- une unité de filtration (5) pour séparer l'au moins un agent stabilisant chargé au moins en partie en polyphénol, et
- une conduite d'évacuation (6) pour évacuer le liquide stabilisé appauvri en l'au moins un type de polyphénol,
dans lequel le dispositif est réalisé de manière à pouvoir effectuer le procédé des revendications 1 à 14 en tant qu'un processus en ligne continu dans un processus en cours de la fabrication et du transvasement de boissons.

16. Dispositif selon la revendication 15, **caractérisé en ce qu'**en supplément une unité de commande est prévue, dans lequel l'unité de commande est réalisée pour être reliée de manière communicante à l'unité de comparaison et d'évaluation (3) et pour commander l'unité de dosage (4).

17. Dispositif selon la revendication 15 ou 16, **caractérisé en ce qu'**en supplément un autre dispositif de mesure (2') est prévu.
